(19) **Europäisches Patentamt / European Patent Office / Office européen des brevets**

(11) **EP 3 904 497 B1**

(12) **EUROPÄISCHE PATENTSCHRIFT**

(45) Veröffentlichungstag und Bekanntmachung des Hinweises auf die Patenterteilung:
**27.03.2024   Patentblatt 2024/13**

(21) Anmeldenummer: **19905217.6**

(22) Anmeldetag: **05.12.2019**

(51) Internationale Patentklassifikation (IPC):
*G01N 33/52* (2006.01)   *C12M 1/34* (2006.01)
*C12Q 1/04* (2006.01)   *C12Q 1/58* (2006.01)

(52) Gemeinsame Patentklassifikation (CPC):
**C12M 41/46; C12Q 1/04; C12Q 1/58; G01N 33/526**

(86) Internationale Anmeldenummer:
**PCT/RU2019/000906**

(87) Internationale Veröffentlichungsnummer:
**WO 2020/139142 (02.07.2020 Gazette 2020/27)**

(54) **TESTSYSTEM ZUR BESTIMMUNG DER ENZYMATISCHEN AKTIVITÄT VON BAKTERIEN AUF AMMONIAK**

TESTING SYSTEM FOR DETERMINING THE ENZYMATIC ACTIVITY OF BACTERIA ON AMMONIA

SYSTÈME DE TEST POUR DÉTERMINER L'ACTIVITÉ DE FERMENTATION DE BACTÉRIES PAR RAPPORT À L'AMMONIAC

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.12.2018   RU 2018146363**

(43) Veröffentlichungstag der Anmeldung:
**03.11.2021   Patentblatt 2021/44**

(73) Patentinhaber: **"Association of Medicine and Analytics" Co. Ltd**
**(AMA Co. Ltd)**
**Sankt-Peterburg, 199034 (RU)**

(72) Erfinder:
• **DMITRIENKO, Marina Alexandrovna**
  **Sankt-Peterburg, 198215 (RU)**
• **AKSENOV, Evgeniy Matveevich**
  **Leningradskaya  oblast', g. Kirovsk 187342 (RU)**
• **DMITRIENKO, Vadim Sergeevich**
  **Sankt-Peterburg, 198215 (RU)**
• **KOLOMINA, Elena Olegovna**
  **Astrakhan', 414040 (RU)**

(74) Vertreter: **Jeck, Anton**
**Jeck, Fleck & Partner mbB**
**Patentanwälte**
**Klingengasse 2**
**71665 Vaihingen/Enz (DE)**

(56) Entgegenhaltungen:
EP-A1- 3 009 519          EP-A1- 3 009 519
WO-A2-2011/112106         DE-A1-102009 046 430
DE-T2- 69 330 515         RU-C9- 2 630 651
RU-C9- 2 630 651          US-A- 5 314 804
US-A1- 2003 077 680       US-A1- 2012 094 371

**Beschreibung**

Die Erfindung bezieht sich auf die Medizin, insbesondere auf Testsysteme zur Bestimmung der enzymatischen Aktivität von Bakterien nach Ammoniak, und zwar der enzymatischen Aktivität von Bakterien, die Parodontal- und Magenerkrankungen sowie Erkrankungen des Urogenitalsystems beim Menschen und bei Tieren verursachen.

Die enzymatische Aktivität von Bakterien findet eine weite Verwendung in der Medizin als Indikator für eine Infizierung mit verschiedenen Bakterien. Ein Beispiel ist der Nachweis von *H.pylori* im Magen des Menschen durch Urease-Aktivität. In der Fachliteratur gibt es Hinweise, dass je nach untersuchtem Biomaterial auch andere Bakterien mit Enzymen nachgewiesen werden können, die die Reaktion der Substratspaltung mit Freisetzung von Ammoniak katalysieren. So kann das orale Mikrobiom viele Mikroorganismen enthalten. Diese Mikroorganismen haben eine Urease-Aktivität und können eine Entzündung verursachen. Dazu gehören beispielsweise *Streptococcus sanguis, Bacteroides melaninogenicus, Prevotella melaninogenica, Actinomyces viscosus.* Darüber hinaus wird in den letzten Jahren eine wichtige Rolle von lokalen Infektionen durch Urease erzeugende Mikroorganismen (*Proteus mirabilis, Corynebacterium urealyticum, Ureaplasma urealyticum* etc.) bei der Entstehung von Ammonium- und Calciumphosphatsteinen ("infektiöse Urolithiasis") in Betracht gezogen. Die invasiven und zytotoxischen Eigenschaften pathogener Bakterienstämme führen zu einer Läsion der Schleimhautoberflächen des Harntrakts. Die Urease-Produktion trägt zu einer Alkalisierung des Urins und zur Bildung von Struvit-Konkrementen bei. Neben der Nierensteinbildung kann *Ureaplasma urealyticum* die Ursache für entzündliche Erkrankungen des Fortpflanzungssystems sein. Dieses kann nach Urease-Aktivität differentialdiagnostisch nachgewiesen werden. Ein weiteres sexuell übertragbares Bakterium, *Mycoplasma,* kann auf ähnliche Weise nachgewiesen werden. Bei diesem Mikroorganismus erfolgt die Bestimmung anhand eines Komplexes von Enzymen, die Arginin zu Ammoniak abbauen.

Bekannt ist das Verfahren zur Bestimmung der Urease-Aktivität ("Urologie und Nephrologie", 1997, Nr. 4, S. 13 - 14). Das Verfahren besteht darin, dass in zwei Reagenzgläser je 2 ml des Untersuchungsurins gegeben werden. Ins Reagenzglas Nr. 1 werden 0,4 ml von 10%igem Calciumchlorid zugegeben und durchgemischt. Danach wird die Extinktion (optische Dichte) in Bezug auf Wasser bei 691 nm mit einem KFK-3 (Photometer) gemessen. Ins Reagenzglas Nr. 2 werden 0,4 ml destilliertes Wasser zugegeben. Die beiden Reagenzgläser werden eine Stunde lang bei 37°C thermostatisiert. Die Reagenzgläser Nr. 1 und Nr. 2 werden unmittelbar nach der Thermostatisierung unter den gleichen Bedingungen verwendet. Der nach den Berechnungen erhaltene Wert der Urease-Aktivität wird mit dem Normwert (0 bis 50 mmol/l) verglichen. Wird der Normwert überschritten, so deutet dies auf das Vorhandensein einer Urease bildenden Mikroflora hin. Die Urease bildende Mikroflora schafft günstige Bedingungen für die Kristallbildung. Außerdem fördern die genannten günstigen Bedingungen das Steinwachstum. Dies kann zu einer Urolithiasis führen.

Bekannt ist die Verwendung von Mikrotiterplatten zur Probenvorbereitung der zu untersuchenden biologischen Flüssigkeit (Patent RU 2521201 "Verfahren zur Früherkennung von Harnwegsinfektionen bei Kindern im Alter von 3 bis 7 Jahren", IPC G01N 33/52, veröffentlicht am 27.06.2014). Bei der Durchführung des Verfahrens werden Urinproben in den Näpfchen (Kavitäten) der Mikrotiterplatte vorbereitet. Die Inhalte der Näpfchen der Mikrotiterplatte werden durchgemischt. Anschließend werden die Werte der optischen Dichte mittels photometrischer Methode bei einer Wellenlänge von 620 nm mit einem Lesegerät (Plate-Reader) gemessen. Die Mikrotiterplatte wird mit Folie verschlossen. Die Inhalte der Näpfchen werden eine Stunde lang bei 37°C thermostatisiert. Danach werden die Inhalte der Näpfchen erneut durchgemischt. Anschließend wird die optische Dichte der Urinproben gemessen. Dann wird eine Kalibrierkurve gezeichnet und die Urease-Aktivität des Urins in U/l nach der Formel

$$UA(U/l) = \Delta D * 11655 \text{ berechnet,}$$

wobei

UA(U/l) - Urease-Aktivität in (U/l);
$\Delta D$ - die Differenz zwischen den optischen Dichten der Testprobe und der Kontrollprobe nach der Thermostatisierung,
11655 - der Umrechnungsfaktor für die Umrechnung auf Urease-Aktivität sind.

Wenn der Wert der Urease-Aktivität im Urin höher als 1621,73 U/l ist, gilt das Kind als gefährdet für die Entwicklung einer Harnwegsinfektion und wird einer entsprechenden Risikogruppe zugerechnet.

Im Verfahren gemäß dem Patent RU 2597777 ("Verfahren zur Bestimmung der Urease-Aktivität der Mundflüssigkeit zum Screening einer von Urease-positivem Mikrobiota besiedelten Mundhöhle", IPC G01N 33/50, G01N 33/52, veröffentlicht am 20.06.2016) werden ebenfalls Mikrotiterplatten zur Vorbereitung der zu untersuchenden biologischen Flüssigkeit verwendet. Zu diesem Zweck wurde die Bestimmung der Urease-Aktivität wie folgt durchgeführt. In eines der Näpfchen der Mikrotiterplatte wurde wässrige Harnstofflösung mit Phosphatpuffer gegeben. In die beiden anderen Näpfchen wurden wässrige Harnstofflösungen mit Phosphatpuffer zugegeben, die Urease mit einer bekannten Konzentration von 5 bzw. 10 U/l enthielten. In die restlichen Näpfchen wurde wässrige Harnstofflösung mit Phosphatpuffer und Proben der Mundflüssigkeit zugegeben.

Danach wurden Phenol/Nitroprussid-Reagens und Hypochlorit in alle Näpfchen gegeben. Die optische Dichte wurde dann auf einem Lesegerät (Plate-Reader) bei einer Wellenlänge von 546 nm gemessen. Die Konzentration der Urease in den Mundflüssigkeitsproben wurde in U/l berechnet. Wenn der Wert der Urease-Aktivität höher als 15,85±2,11 U/l war, wurde eine Besiedlung der Mundhöhle mit Urease-positivem Mikrobiota festgestellt. Das Ergebnis ermöglicht es, die Notwendigkeit einer antimikrobiellen Therapie zur Vorbeugung der Bildung eines harten Zahnbelags und von entzündlichen Parodontalerkrankungen festzustellen.

Bekannt ist ein Set zur Labordiagnostik von Infektionen (Patent RU 2553548 "Set zur Labordiagnostik von durch *Mycoplasma hominis* und *Ureaplasma urealyticum* verursachten Infektionen", IPC C12Q 1/02, veröffentlicht am 20.06.2015). Dieses Set kann zur Züchtung und Identifizierung von urogenitalen Mykoplasmen, insbesondere *Mycoplasma hominis* und *Ureaplasma urealyticum,* zur Labordiagnostik von urogenitalen Mykoplasmosen durch Nachweis von Mykoplasmen im klinischen Material, zur semiquantitativen Bestimmung des Erregertiters sowie zur Bestimmung ihrer Antibiotikaresistenz eingesetzt werden.

Ein solches Set enthält ein lyophilisiertes Transportmedium für die Lagerung des zu untersuchenden biologischen Materials, ein lyophilisiertes Nährmedium für den Nachweis von *Mycoplasma hominis* und/oder *Ureaplasma urealyticum* sowie Teststreifen für die Identifizierung von *Mycoplasma hominis* und *Ureaplasma urealyticum,* zu einer semiquantitativen Bestimmung des Erregertiters und zur Bestimmung der Empfindlichkeit von *Mycoplasma hominis* und *Ureaplasma urealyticum* gegen Antibiotika. Das Set enthält drei Typen von Teststreifen. Der erste davon ist zur Identifizierung von *Mycoplasma hominis* und *Ureaplasma urealyticum* und zu einer semiquantitativen Bestimmung ihres Titers bestimmt. Dabei enthält ein Teil der Näpfchen des ersten Teststreifentyps, die für den Nachweis und die Bestimmung des Titers von *Mycoplasma hominis* geeignet sind, Arginin, Brillantblau und Clarithromycin. Der andere Teil der Kavitäten dieses Teststreifens, die für den Nachweis und die Bestimmung des Titers von *Ureaplasma urealyticum* vorgesehen sind, enthalten Harnstoff und Lincomycin. Der zweite Teststreifentyp ist für die Bestimmung der Antibiotikaresistenz von *Mycoplasma hominis* geeignet. Dieser enthält spezifische Reagenzien für den Nachweis von *Mycoplasma hominis,* und zwar Arginin, Brillantblau und Clarithromycin. Außerdem enthält dieser Teststreifentyp acht Antibiotika: Doxycyclin, Josamycin, Midecamycin, Ofloxacin, Ciprofloxacin, Sparfloxacin, Moxifloxacin, Clindamycin. Die Antibiotika sind in gleicher Konzentration in die Näpfchen der Teststreifen sorbiert. Der dritte Teststreifentyp ist für die Bestimmung der Antibiotikaresistenz von *Ureaplasma urealyticum* vorgesehen. Er enthält spezifische Reagenzien für den Nachweis von *Ureaplasma urealyticum,* und zwar Harnstoff und Lincomycin sowie acht Antibiotika: Doxycyclin, Josamycin, Clarithromycin, Erythromycin, Roxithromycin, Azithromycin, Ofloxacin und Sparfloxacin. Die Antibiotika werden in gleicher Konzentration in die Näpfchen der Teststreifen sorbiert. Die Verwendung des Sets ermöglicht es, *Mycoplasma hominis* und *Ureaplasma urealyticum* gleichzeitig nachzuweisen, ihren Titer zu beurteilen und die Empfindlichkeit gegenüber unterschiedlichen Spektren von Antibiotika zu bestimmen. Dabei werden für *Mycoplasma hominis* und für *Ureaplasma urealyticum* unterschiedliche Antibiotika eingesetzt.

Die oben dargestellten Testsysteme zur Bestimmung einer enzymatischen Aktivität von Bakterien nach Ammoniak werden unter Laborbedingungen (*in vitro*) eingesetzt. Diese setzen die Benutzung von zusätzlichen Geräten wie Photokolorimeter oder Thermostat voraus. Dies erfordert den Einsatz von qualifiziertem Personal.

Bekannt sind Testsysteme zur Bestimmung der enzymatischen Aktivität von Bakterien nach Ammoniak zum Nachweis von *Helicobacter pylori* (z.B. WO2011112106, IPC C12Q 1/58, veröffentlicht am 15.09.2011; US2010028937, IPC C12M1/00; C12Q1/02, veröffentlicht am 04.02.2010; WO9319200, IPC C12Q1/00; C12Q1/04, veröffentlicht am 30.09.1993). Bei diesen Testsystemen handelt es sich um eine mehrschichtige und mehrteilige Testvorrichtung. Die Testvorrichtung besteht im Wesentlichen aus einem Substratelement. Das Substratelement enthält Harnstoff zur Bestimmung einer Urease-Aktivität des zu untersuchenden biologischen Materials und ein Diffusionselement. Das Diffusionselement dient dazu, Fehlreaktionen und einen Durchtritt von Ammoniak zum Indikatorelement zu verhindern. Anhand des Farbumschlags des Indikatorelements kann das Ergebnis beurteilt werden.

Solchen Testsystemen liegen jedoch konstruktive Lösungen zugrunde. Die konstruktiven Lösungen ermöglichen die Untersuchung von biologischem Material, das eine geringe Menge an flüssiger Phase (Biopsat) enthält. Das Vorhandensein eines Diffusionselements verringert die Empfindlichkeit solcher Testsysteme und erlaubt nicht die Bestimmung der Urease-Aktivität anderer Bakterien.

Aus der US 2012/0094371 ist ein dreischichtiges Testsystem zur Indikation einer Helicobacter-pylori Aktivität bekannt, bei welcher eine hydrophile Substratschicht von einer Indikatorschicht über eine hydrophobe Trennschicht separiert ist.

Die RU 2 630 651 C9 ist ein dreischichtiges Testsystem zur Indikation einer Helicobacter-pylori Aktivität bekannt, bei welcher eine hydrophile Substratschicht von einer Indikatorschicht über eine hydrophobe Trennschicht separiert ist.

Aus der DE 693 30 515 T2 geht ein dreischichtiges Testsystem zur Indikation einer Helicobacter-pylori Aktivität hervor, bei welchem auf einer Indikatorschicht eine hydrophobe Membran angeordnet ist, welcher eine hydrophile substrattragende Schicht zustellbar ist.

Als nächstliegend der beanspruchten Lösung wird die technische Lösung gemäß dem Patent EP3009519 ("Test device, reactive element and arrangement for urease activity" IPC B01L3/00, C12Q1/58, G01N33/487, veröffentlicht am 14.10.2014) angesehen. Diese Lösung enthält eine Unterlage mit mindestens einem Näpfchen (einer Kavität). In dem Näpfchen ist ein reaktives Element angeordnet. Das reaktive Element besteht aus zwei Schichten, einer Indikatorschicht und einer empfindlichen Schicht. Die Letztere dient zur Anordnung des zu untersuchenden biologischen Materials, während die Indikatorschicht am Boden des Näpfchens mit einem Sichtfenster angeordnet ist.

Um die enzymatische Aktivität von Bakterien nach Ammoniak zu bestimmen, wird das zu untersuchende biologische Material auf die empfindliche Schicht des reaktiven Elements angeordnet. Danach erfolgt eine Reaktion der Spaltung von Harnstoff der empfindlichen Schicht. Der endgültige Farbumschlag der Indikatorschicht kann durch das Sichtfenster beobachtet werden. Daran wird über das Vorhandensein einer Urease-Aktivität einer Bakterie wie z. B. *Helicobacter pylori* beurteilt.

Die konstruktive Lösung dieses Testsystems ermöglicht die Untersuchung von biologischem Material, das eine geringe Menge an flüssiger Phase (z. B. Biopsat) enthält, da flüssiges Untersuchungsmaterial bis zur Indikatorschicht durchdringen wird. Dies wird zu einem unzuverlässigen Ergebnis führen. Dadurch wird der Anwendungsbereich des Testsystems eingeschränkt.

Außerdem zeichnet sich dieses Testsystem durch eine beträchtliche Reaktionszeit aus, da die Schichten des reaktiven Elements frei im Näpfchen angeordnet sind. Dies führt zu einem Verlust von freigesetztem Ammoniak, was wiederum die Zeit der Bestimmung einer enzymatischen Aktivität der Bakterien erhöht.

Die Aufgabe der beanspruchten Erfindung besteht darin, ein Testsystem zur Bestimmung einer enzymatischen Aktivität von Bakterien nach Ammoniak zu entwickeln, mit dem ein technischer Effekt erzielt werden kann, und zwar die Verkürzung der Zeit für die Bestimmung einer Aktivität von enzymatischen Reaktionen und die Erweiterung des Anwendungsbereiches mittels Erweiterung der Typen der Untersuchungsproben.

Der Grundgedanke der Erfindung besteht darin, dass das Testsystem zur Bestimmung einer enzymatischen Aktivität der Bakterien nach Ammoniak eine Unterlage mit einem Sichtfenster zur Einsicht auf die Ergebnisse enthält. Auf der Unterlage ist ein reaktives Element angeordnet. Das reaktive Element besteht aus einer Indikatorschicht und einer empfindlichen Schicht. Die Indikatorschicht überlagert das Sichtfenster. Die empfindliche Schicht enthält ein Substrat zum Nachweis einer enzymatischen Aktivität. Gemäß der Erfindung befindet sich zusätzlich auf der Unterlage eine Fixierfolie mit einer Öffnung. Das fixierende Blatt ist vorgesehen, um das zu untersuchende biologische Material auf der empfindlichen Schicht des reaktiven Elements zu platzieren. Die Größe des fixierenden Blatts stellt die Fixierung der empfindlichen Schicht sicher. Auf dem fixierenden Blatt ist eine Deckfolie) aufgelegt. Das Deckblatt ist auf der dem fixierenden Blatt zugewandten Seite mit einer wiederverschließbaren Befestigungsbeschichtung versehen. Die einander zugewandten Oberflächen der Unterlage und des fixierenden Blatts sind dabei mittels einer Klebeschicht verbunden. Das reaktive Element ist so aufgebaut, dass die Fläche der empfindlichen Schicht größer ist als die Fläche der undurchsichtigen Indikatorschicht. Die Indikatorschicht enthält einen ammoniakempfindlichen Indikator. Die empfindliche Schicht ist als eine halbdurchlässige hydrophobe Membran ausgebildet. Dabei sind die Unterlage, das fixierende Blatt und das Deckblatt aus einem undurchlässigen, hydrophoben Material ausgebildet. Die Dicken der Unterlage, des fixierenden Blatts und des Deckblatts sowie die Dicke jeder der Schichten des reaktiven Elements liegen im Bereich von 50 bis 300 μm.

Die Indikatorschicht des reaktiven Elements kann aus einem hydrophilen polymeren Material ausgebildet sein.

Das hydrophile polymere Material wird aus der folgenden Reihe ausgewählt: Papier, Polyesterfasern oder Polyvinylalkoholfolie (PVA-Folie).

Der ammoniakempfindliche Indikator wird aus der folgenden Reihe ausgewählt: pH-Indikatoren, Neßler-Reagens, Chrom(II)-chlorid.

Das undurchlässige hydrophobe Material, aus dem die Unterlage, das fixierende Blatt und das Deckblatt ausgebildet sind, wird aus der folgenden Reihe ausgewählt: Polypropylen, Polyvinylchlorid, Polyester.

Die empfindliche Schicht des reaktiven Elements, die eine halbdurchlässige hydrophobe Membran darstellt, wird entweder aus Polyethylen, Polytetrafluorethylen oder Polypropylen ausgeführt.

Das Substrat zum Nachweis einer enzymatischen Aktivität der empfindlichen Schicht des reaktiven Elements wird aus der folgenden Reihe ausgewählt: Harnstoff, Arginin, Ornithin.

Die wiederverschließbare Befestigungsbeschichtung ist aus einem Einkomponentenklebstoff ausgebildet, der aus der folgenden Reihe ausgewählt wird: Polyurethan, Acetophenon, Polyvinylacetat, Polyisobutylen.

Die Klebeschicht, die die einander zugewandten Oberflächen der Unterlage und des fixierenden Blatts verbindet, besteht aus einem Einkomponentenklebstoff. Dieser wird aus der folgenden Reihe ausgewählt: Polyurethan, Polyvinylacetat, Isobornylacrylat, Polyisobutylen.

Das Deckblatt kann mit einer bogenförmigen Vorderkante in Form einer Lasche auf einer der Seiten ausgeführt werden.

Das Deckblatt kann auf einer Seite mit einer Schicht Schmelzklebstoff befestigt werden. Diese Seite ist der bogenförmigen Vorderkante gegenüberliegend.

Das Deckblatt kann entweder transparent oder mit einem transparenten Bereich ausgeführt sein. Der transparente Bereich entspricht der Größe der Öffnung im fixierenden Blatt.

Der Hauptunterschied des beanspruchten Testsystems besteht darin, dass das fixierende Blatt und die Unterlage sowie das dazwischen angeordnete reaktive Element die Bildung eines Raumes für Ammoniak ermöglichen. Das fixierende Blatt und die Unterlage sind dabei durch eine Klebeschicht fest miteinander verbunden. Ammoniak wird im Zuge der Zielreaktionen freigesetzt. Die Zielreaktionen umfassen eine Spaltungsreaktion zum Aufspalten des Substrats der empfindlichen Schicht sowie eine Interaktion zwischen Ammoniak und dem Indikator. Je nach der zu bestimmenden Bakterie können als Substrat der empfindlichen Schicht Harnstoff, Arginin oder Ornithin dienen. Aufgrund einer geringen Dicke des fixierenden Blatts, der Unterlage sowie der Schichten des reaktiven Elements ist die Raumgröße minimal hinreichend für eine schnelle Wechselwirkung zwischen Ammoniak und dem Indikator in diesem Raum. Dadurch kann die Reaktionszeit auf 5 Minuten reduziert werden, während sie bei den ähnlichen Lösungen bei 15 Minuten beginnt. Die oben genannten Dicken des fixierenden Blatts, der Unterlage sowie der Schichten des reaktiven Elements reichen von 50 bis 300 $\mu$m. Der Dickenbereich für die Dicken des fixierenden Blatts, der Unterlage und der Schichten des reaktiven Elements ist durch Folgendes bedingt. Material mit einer Dicke bis 50 $\mu$m weist eine unzureichende Festigkeit auf. Material, das dicker als 300 $\mu$m ist, wird einen zu großen Raum bilden und die Reaktion zwischen Ammoniak und dem Indikator verlangsamen. Das beanspruchte Testsystem bietet die Möglichkeit, sowohl halbfestes (Biopsat) als auch flüssiges biologisches Untersuchungsmaterial (Urin, Speichel) zu analysieren. Dies ist auch durch den Aufbau des Testsystems gewährleistet. Der Schutz der Indikatorschicht des reaktiven Elements vor dem Durchdringen der zu untersuchenden Flüssigkeit wird gewährleistet durch hydrophobe Eigenschaften des Materials, aus dem die empfindliche Schicht des reaktiven Elements ausgeführt ist, sowie durch die Voraussetzung, dass die Fläche der empfindlichen Schicht größer sein muss als die Fläche der Indikatorschicht. Auf diese Weise kann die Indikatorschicht, die auf der Unterlage platziert ist, von oben mit einer empfindlichen Schicht abgedeckt werden. Dies bietet Schutz vor Kontakt mit der zu analysierenden Flüssigkeit. Das fixierende Blatt ist aus einem hydrophoben, undurchlässigen Material gefertigt. Dieses Material hilft, überschüssige Flüssigkeit von der Oberfläche abzuleiten. Außerdem verhindert das genannte Material, dass die genannte Flüssigkeit in das reaktive Element eindringt. Das Vorhandensein eines Deckblatts im Testsystem stellt sicher, dass das Testsystem luftdicht ist. Außerdem ist das Trocknen des zu untersuchenden biologischen Materials im Laufe der Untersuchung ausgeschlossen. Das beanspruchte Testsystem bedarf keiner zusätzlichen Vorbereitungen vor der Untersuchung, da alle notwendig hinreichenden Substanzen (Indikator, Substrat) hermetisch im Testsystem eingeschlossen sind.

Die Erfindung wird anhand von Zeichnungen näher erläutert. Es zeigen:

      Fig. 1 - eine Querschnittsansicht des Testsystems,
      Fig. 2 - eine vereinfachte Explosionszeichnung des Testsystems (Axonometrie),
      Fig. 3 - ein Testsystem im montierten Zustand (Axonometrie).

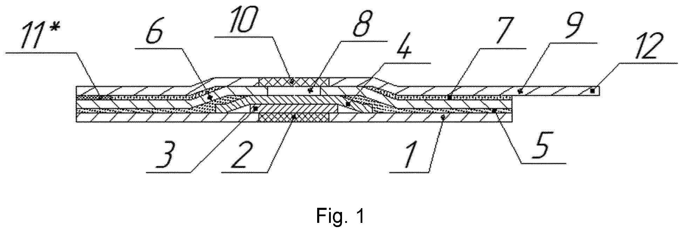

Das Testsystem (Fig. 1) besteht aus einer Unterlage 1 mit einem Sichtfenster 2 zur Einsicht auf die Ergebnisse. Auf der Unterlage 1 ist ein zweischichtiges reaktives Element angeordnet, wobei die Indikatorschicht 3 des reaktiven Elements das Sichtfenster 2 überlagert. Die empfindliche Schicht 4 des reaktiven Elements ist auf der Indikatorschicht 3 des reaktiven Elements angeordnet. Die Fläche der empfindlichen Schicht 4 des reaktiven Elements ist größer als die Fläche der Indikatorschicht 3 des reaktiven Elements. Auf der Unterlage 1 mit dem reaktiven Element ist eine Fixierfolie 6 mittels einer Klebeschicht 5 zur Anordnung des zu untersuchenden biologischen Materials 8 auf der empfindlichen Schicht des reaktiven Elements befestigt. Das fixierende Blatt 6 weist eine Öffnung auf. Die Abmessungen des fixierenden Blatts stellt die Fixierung der empfindlichen Schicht 4 des reaktiven Elements sicher. Auf dem fixierenden Blatt 6 ist eine Deckfolie 9 mit einem transparenten Bereich 10 mittels einer wiederverschließbaren Befestigungsbeschichtung 7 befestigt. Das Deckblatt 9 ist auf einer der Seiten mit einer Schicht 11 Schmelzklebstoff fixiert. Auf der anderen Seite besitzt es eine Lasche 12 zum Greifen beim Abziehen und Decken des Deckblatts 9.

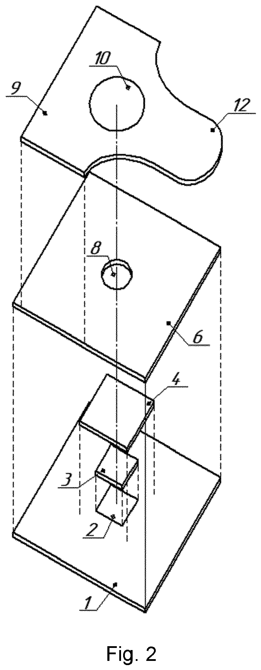

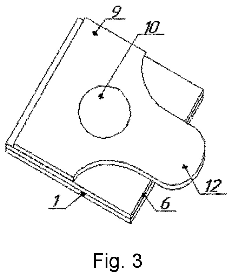

Der Aufbau des Prüfsystems ist schichtweise in Fig. 2 dargestellt. Hier sind die Schichten des Testsystems ohne die Klebeschicht und die wiederverschließbare Befestigungsbeschichtung in einer axonometrischen Ansicht gezeigt. Im zusammengebauten Zustand ist das Testsystem in Fig. 3 dargestellt.

Um die enzymatische Aktivität der Bakterien nach Ammoniak zu bestimmen, wird das Deckblatt 9 anhand der Lasche 12 abgezogen . Dann wird eine Probe des zu untersuchenden biologischen Materials 8 in das Fenster des fixierenden Blatts 6 auf der empfindlichen Schicht 4 des reaktiven Elements angeordnet. Danach wird die angeordnete Probe des zu untersuchenden biologischen Materials mit dem Deckblatt 9 dicht gedeckt. Anschließend wird das Testsystem umgedreht, damit die Reaktion durch das Sichtfenster 2 zur Einsicht auf die Ergebnisse beobachtet werden kann. Nach 5 Minuten wird der Farbumschlag der Indikatorschicht 3 des reaktiven Elements im Sichtfenster 2 beurteilt.

Die enzymatische Aktivität von Bakterien, die Parodontalerkrankungen verursachen, wird durch die Farbänderung der Indikatorschicht des reaktiven Elements bestimmt. Dafür werden auf der Indikatorschicht die zu untersuchenden Proben einer gemischten Mundflüssigkeit angeordnet. Falls in den zu untersuchenden Proben Ureasepositive Bakterien vorhanden sind, erscheint innerhalb von 5 Minuten auf der Oberfläche der Indikatorschicht des reaktiven Elements ein roter oder ein himbeerfarbener Farbfleck.

Die enzymatische Aktivität von Bakterien, die eine Erkrankung wie Urolithiasis verursachen, wird durch eine Veränderung der Farbe der Indikatorschicht des reaktiven Elements bestimmt, indem die zu untersuchenden Urinproben auf der Indikatorschicht angeordnet werden. Falls in den zu untersuchenden Proben die Bakterien *Proteus mirabilis* und *Corynebacterium urealyticum* vorhanden sind, erscheint auf der Oberfläche der Indikatorschicht des reaktiven Elements ein roter oder ein himbeerfarbener Farbfleck.

Die enzymatische Aktivität von Bakterien, die gynäkologische oder urologische Erkrankungen verursachen, wird wie folgt bestimmt: Falls in den zu untersuchenden Proben die Bakterien *Mycoplasma* und *Ureaplasma* vorhanden sind, erscheint auf der Oberfläche der Indikatorschicht des reaktiven Elements innerhalb von 5 Minuten ein roter oder ein himbeerfarbener Farbfleck. Proben zur Untersuchung können sein:

- für Männer - ein Harnröhrenabstrich;
- für Frauen - Vaginalausfluss oder endozervikaler Schleim.

Als Beispiel für eine konkrete Umsetzung des beanspruchten Testsystems kann der folgende Aufbau eines Testsystems zur Bestimmung einer enzymatischen Aktivität von *Mycoplasma* nach Ammoniak in einer Probe des zu untersuchenden biologischen Materials, und zwar des endozervikalen Schleims, dienen.

Die Unterlage, das fixierende Blatt und das Deckblatt sind in Form von Quadraten mit einer Größe von 25×25 mm aus Polypropylen gefertigt. Die Dicke von Polypropylen beträgt 250 $\mu$m.

Die Indikatorschicht des reaktiven Elements ist aus WHATMAN-Filterpapier TYP 1 in Form eines Quadrats mit einer Größe von 5×5 mm und einer Dicke von 100 $\mu$m gefertigt. Das Filterpapier ist mit Phenolrot als pH-Indikator imprägniert.

Die empfindliche Schicht des reaktiven Elements ist als eine Membran aus Polyethylenterephthalat ausgeführt. Auf die Membran ist Arginin aufgetragen. Arginin dient als Substrat für die zu bestimmende Aktivität eines Komplexes von Enzymen, die Arginin zu Ammoniak abbauen. Die empfindliche Schicht ist in Form eines Quadrats mit einer Größe von 10×10 mm und einer Dicke von 150 $\mu$m gefertigt.

Das fixierende Blatt hat eine Öffnung mit einem Durchmesser von 5 mm. Diese Öffnung dient zur Anordnung des zu untersuchenden biologischen Materials, und zwar des endozervikalen Schleims, auf der empfindlichen Schicht des reaktiven Elements.

Die einander zugewandten Oberflächen der Unterlage und des fixierenden Blatts sind mit Hilfe einer Klebeschicht verbunden. Die Klebeschicht ist mit einem Einkomponentenklebstoff auf Basis von Polyvinylacetat KIWOTHERM D 123 ausgeführt. Das Deckblatt ist mit dem fixierenden Blatt mittels des Klebstoffs LOCTITE DURO - TAK 4003 geklebt und hat einen transparenten Bereich. Der transparente Bereich entspricht der Größe der Öffnung im fixierenden Blatt von 5 mm.

Das Sichtfenster der Unterlage ist in Form eines Quadrats mit einer Größe von 5×5 mm ausgeführt.

Das Deckblatt ist auf der einen Seite mit einer Lasche zum einfachen Öffnen des Testsystems versehen. Auf der anderen Seite ist es mit einer Schicht LUNAMELT HS-3500-Schmelzklebstoff zur sicheren Fixierung des Deckblatts befestigt.

Unter Beibehaltung aller konstruktiven Maße bzw. deren Veränderung in akzeptablen Grenzen ermöglicht die Substratsubstitution, und zwar von Arginin zu Harnstoff oder Ornithin, die Untersuchung anderer Bakterien wie z. B. *Proteus mirabilis, Corynebacterium urealyticum, Helicobacter pylori, Ureaplasma urealyticum* etc. Die Untersuchung erfolgt dabei mit verschiedenen Arten von Untersuchungsproben, nämlich mit flüssigen, halbfesten und festen Proben.

Somit ermöglicht das beanspruchte Testsystem eine Erweiterung des Anwendungsbereichs durch die Erweiterung der Typen von Untersuchungsproben und die Verkürzung der Zeit für die Bestimmung der Aktivität von enzymatischen Reaktionen.

**Patentansprüche**

1. Testsystem zur Bestimmung einer enzymatischen Aktivität von Bakterien nach Ammoniak umfasst eine Unterlage (1) mit einem Sichtfenster (2) zur Einsicht auf die Ergebnisse. Auf der Unterlage (1) ist ein reaktives Element angeordnet. Das reaktive Element besteht aus einer Indikatorschicht (3) und einer empfindlichen Schicht (4). Die Indikatorschicht (3) überlagert das Sichtfenster (2). Die empfindliche Schicht (4) enthält ein Substrat zum Nachweis einer enzymatischen Aktivität.

   Das Testsystem ist **dadurch gekennzeichnet, dass** auf der Unterlage (1) zusätzlich ein fixierendes Blatt (6) angeordnet ist. Das fixierende Blatt ist für die Anordnung des zu untersuchenden biologischen Materials auf der empfindlichen Schicht des reaktiven Elements bestimmt (4). Das fixierende Blatt (6) weist eine Öffnung auf. Die Grö-ße des fixierenden Blatts (6) stellt die Befestigung der empfindlichen Schicht (4) sicher. Auf dem fixierenden Blatt ist ein Deckblatt (9) angeordnet. Das Deckblatt (9) ist mit einer wiederverschließbaren Befestigungsbeschichtung auf der dem fixierenden Blatt (6) zugewandten Seite bedeckt. Dabei werden die einander zugewandten Oberflächen der Unterlage (1) und des fixierenden Blatts (6) mittels einer Klebeschicht (5) miteinander verbunden. Das reaktive Element ist so organisiert, dass die Fläche der empfindlichen Schicht (3) größer ist als die Fläche der undurchsichtigen Indikatorschicht. Die empfindliche Schicht (4) ist als eine halbdurchlässige hydrophobe Membran ausgebildet. Die Indikatorschicht (3) enthält einen ammoniakempfindlichen Indikator. Die Unterlage (1), das fixierende Blatt (6) und das Deckblatt (9) sind aus einem undurchlässigen, hydrophoben Material gefertigt. Die Dicken der Unterlage (1), des fixierenden Blatts (6) und des Deckblatts (9) sowie die Dicke jeder der Schichten des reaktiven Elements legen im Bereich von 50 bis 300 μm.

2. Testsystem zur Bestimmung einer enzymatischen Aktivität von Bakterien nach Ammoniak nach Anspruch 1,
   **dadurch gekennzeichnet,**
   **dass** die Indikatorschicht (3) des reaktiven Elements aus einem hydrophilen polymeren Material ausgebildet ist.

3. Testsystem zur Bestimmung einer enzymatischen Aktivität von Bakterien nach Ammoniak nach Anspruch 2,
   **dadurch gekennzeichnet,**
   **dass** das hydrophile polymere Material Papier, Polyesterfasern oder Polyvinylalkoholfolie (PVA-Folie) darstellt.

4. Testsystem zur Bestimmung einer enzymatischen Aktivität von Bakterien nach Ammoniak nach Anspruch 2,
   **dadurch gekennzeichnet,**
   **dass** der ammoniakempfindliche Indikator ein pH-Indikator, ein Neßler-Reagens oder Chrom(II)-chlorid darstellt.

5. Testsystem zur Bestimmung einer enzymatischen Aktivität von Bakterien nach Ammoniak nach Anspruch 1,
   **dadurch gekennzeichnet,**
   **dass** das undurchlässige, hydrophobe Material, aus dem die Unterlage (1), das fixierende Blatt (6) und das Deckblatt (7) gefertigt sind, aus Polypropylen, Polyvinylchlorid oder Polyester besteht.

6. Testsystem zur Bestimmung einer enzymatischen Aktivität von Bakterien nach Ammoniak nach Anspruch 1,
   **dadurch gekennzeichnet,**
   **dass** die empfindliche Schicht (4) des reaktiven Elements, die als eine halbdurchlässige hydrophobe Membran ausgebildet ist, aus Polyethylen, Polytetrafluorethylen oder Polypropylen besteht.

7. Testsystem zur Bestimmung einer enzymatischen Aktivität von Bakterien nach Ammoniak nach Anspruch 1,

**dadurch gekennzeichnet,**
**dass** das Substrat zum Nachweis einer enzymatischen Aktivität der empfindlichen Schicht (4) des reaktiven Elements Harnstoff, Arginin oder Ornithin darstellt.

8. Testsystem zur Bestimmung einer enzymatischen Aktivität von Bakterien nach Ammoniak nach Anspruch 1,
   **dadurch gekennzeichnet,**

   **dass** die wiederverschließbare Befestigungsbeschichtung (9) aus einem Einkomponentenklebstoff ausgeführt ist und
   **dass** der Einkomponentenklebstoff Polyurethan, Acetophenon, Polyvinylacetat oder Polyisobutylen sein kann.

9. Testsystem zur Bestimmung einer enzymatischen Aktivität von Bakterien nach Ammoniak nach Anspruch 1,
   **dadurch gekennzeichnet,**

   **dass** die Klebeschicht (5), mit der die einander zugewandten Oberflächen der Unterlage (1) und des fixierenden Blatts (6) verbunden sind, mit einem Einkomponentenklebstoff ausgeführt ist und
   **dass** der Einkomponentenklebstoff Polyurethan, Polyvinylacetat, Isobornylacrylat oder Polyisobutylen sein kann.

10. Testsystem zur Bestimmung einer enzymatischen Aktivität von Bakterien nach Ammoniak nach Anspruch 1,
    **dadurch gekennzeichnet,**
    **dass** das Deckblatt (9) auf einer der Seiten mit einer bogenförmigen Vorderkante in Form einer Lasche ausgeführt ist.

11. Testsystem zur Bestimmung einer enzymatischen Aktivität von Bakterien nach Ammoniak nach Anspruch 1,
    **dadurch gekennzeichnet,**
    **dass** das Deckblatt (9) auf der der bogenförmigen Vorderkante gegenüberliegenden Seite mittels einer Schicht Schmelzklebstoff befestigt ist.

12. Testsystem zur Bestimmung einer enzymatischen Aktivität von Bakterien nach Ammoniak nach Anspruch 1,
    **dadurch gekennzeichnet,**

    **dass** das Deckblatt (9) entweder transparent ist oder einen transparenten Bereich aufweist und
    **dass** der transparente Bereich dabei der Größe der Öffnung des fixierenden Blatts (6) entspricht.

**Claims**

1. Test system for determining an enzymatic activity of bacteria after ammonia comprises a base (1) with a viewing window (2) for viewing the results. A reactive element is arranged on the base (1). The reactive element consists of an indicator layer (3) and a sensitive layer (4). The indicator layer (3) overlays the viewing window (2). The sensitive layer (4) contains a substrate for detecting enzymatic activity.
   The test system is **characterized in that** a fixing sheet (6) is additionally arranged on the substrate (1). The fixing sheet is intended for the arrangement of the biological material to be tested on the sensitive layer of the reactive element (4). The fixing sheet (6) has an opening. The size of the fixing sheet (6) ensures the attachment of the sensitive layer (4). A cover sheet (9) is arranged on the fixing sheet. The cover sheet (9) is covered with a resealable fixing coating on the side facing the fixing sheet (6). The facing surfaces of the backing (1) and the fixing sheet (6) are bonded together by means of an adhesive layer (5). The reactive element is organized in such a way that the area of the sensitive layer (3) is larger than the area of the opaque indicator layer. The sensitive layer (4) is designed as a semi-permeable hydrophobic membrane. The indicator layer (3) contains an ammonia-sensitive indicator. The base (1), the fixing sheet (6) and the cover sheet (9) are made of an impermeable, hydrophobic material. The thicknesses of the backing (1), the fixing sheet (6) and the cover sheet (9) as well as the thickness of each of the layers of the reactive element are in the range of 50 to 300 μm.

2. Test system for determining an enzymatic activity of bacteria after ammonia according to claim 1, **characterized in that** the indicator layer (3) of the reactive element is formed from a hydrophilic polymeric material.

3. Test system for determining an enzymatic activity of bacteria after ammonia according to claim 2, **characterized in that** the hydrophilic polymeric material is paper, polyester fibers or polyvinyl alcohol film (PVA film).

4. Test system for the determination of an enzymatic activity of bacteria after ammonia according to claim 2, **characterized in that** the ammonia-sensitive indicator is a pH indicator, a Neßler reagent or chromium(II) chloride.

5. Test system for the determination of an enzymatic activity of bacteria after ammonia according to claim 1, **characterized in that** the impermeable, hydrophobic material from which the base (1), the fixing sheet (6) and the cover sheet (7) are made consists of polypropylene, polyvinyl chloride or polyester.

6. Test system for determining an enzymatic activity of bacteria after ammonia according to claim 1, **characterized in that** the sensitive layer (4) of the reactive element, which is formed as a semi-permeable hydrophobic membrane, consists of polyethylene, polytetrafluoroethylene or polypropylene.

7. Test system for determining an enzymatic activity of bacteria after ammonia according to claim 1, **characterized in that** the substrate for detecting an enzymatic activity of the sensitive layer (4) of the reactive element is urea, arginine or ornithine.

8. Test system for the determination of an enzymatic activity of bacteria after ammonia according to claim 1, **characterized in that** the reclosable attachment coating (9) is made of a one-component adhesive and **in that** the one-component adhesive can be polyurethane, acetophenone, polyvinyl acetate or polyisobutylene.

9. Test system for determining an enzymatic activity of bacteria after ammonia according to claim 1, **characterized in that** the adhesive layer (5), with which the mutually facing surfaces of the base (1) and the fixing sheet (6) are bonded, is made with a single-component adhesive and **in that** the one-component adhesive can be polyurethane, polyvinyl acetate, isobornyl acrylate or polyisobutylene.

10. Test system for determining an enzymatic activity of bacteria after ammonia according to claim 1, **characterized in that** the cover sheet (9) is designed on one of the sides with a curved front edge in the form of a flap.

11. Test system for determining an enzymatic activity of bacteria after ammonia according to claim 1, **characterized in that** the cover sheet (9) is attached to the side opposite the curved front edge by means of a layer of hot-melt adhesive.

12. Test system for determining an enzymatic activity of bacteria after ammonia according to claim 1, **characterized in that** the cover sheet (9) is either transparent or has a transparent area and **in that** the transparent area corresponds to the size of the opening of the fixing sheet (6).

**Revendications**

1. Système de test pour la détermination d'une activité enzymatique de bactéries après l'ammoniaque se composant s'une sous-couche (1) avec une fenêtre d'inspection (2) pour avoir l'accès aux résultats. Un élément réactif est disposé au niveau de la sous-couche (1). L'élément réactif se compose d'une couche indicatrice (3) et d'une couche sensible (4). La couche indicatrice (3) superpose la fenêtre d'inspection (2). La couche sensible (4) contient du substrat pour la détection d'une activité enzymatique.

Le système de test est caractérisé de sorte qu'une feuille fixant (6) est disposée en outre sur la sous-couche (1). La feuille fixant est destinée à disposer le matériel biologique à examiner sur la couche sensible de l'élément réactif (4). La feuille fixant (6) présente une ouverture. La taille de la feuille fixant (6) garantit la fixation de la couche sensible (4). Une feuille de couverture (9) est disposée sur la feuille fixant. La feuille de couverture (9) est recouverte d'une partie tournée vers la feuille fixant (6) à l'aide d'un revêtement de fixation refermable. A cet égard, les surfaces en vis-à-vis de la sous-couche (1) et de la feuille fixant (6) sont reliées l'une à l'autre grâce à une couche adhésive (5). L'élément réactif est organisé de sorte que la surface de la couche sensible (3) est plus grande que la surface de la couche indicatrice non transparente. La couche sensible (4) est formée en tant que membrane semi-perméable hydrophobe. La couche indicatrice (3) contient un indicateur sensible à l'ammoniaque. La sous-couche (1), la feuille fixant (6) et la feuille de couverture (9) sont produites à partir d'un matériel imperméable et hydrophobe. Les épaisseurs de la sous-couche (1), de la feuille fixant (6) et de la feuille de couverture (9) ainsi que l'épaisseur de chaque couche de l'élément réactif se situent dans la zone de 50 jusqu'à 300 μm.

2. Système de test pour la détermination d'une activité enzymatique de bactéries après l'ammoniaque selon la revendication 1 est caractérisé de sorte que la couche indicatrice (3) de l'élément réactif est formée d'un matériel hydrophile et polymère.

3. Système de test pour la détermination d'une activité enzymatique de bactéries après l'ammoniaque selon la revendication 2 est caractérisé de sorte que la matériel hydrophile et polymère présente du papier, du polyester ou du film d'alcool polyvinylique (du film PVC).

4. Système de test pour la détermination d'une activité enzymatique de bactéries après l'ammoniaque selon la revendication 2 est caractérisé de sorte que l'indicateur sensible à l'ammoniaque présente un indicateur PH, un réactif Nessler ou un chlorure chrome (II).

5. Système de test pour la détermination d'une activité enzymatique de bactéries après l'ammoniaque selon la revendication 1 est caractérisé de sorte que le matériel imperméable hydrophobe duquel le substrat (1), la lame fixant (6) et la feuille de couverture (7) sont produits, se compose de polypropylène, de polychlorure de vinyle ou de polyester.

6. Système de test pour la détermination d'une activité enzymatique de bactéries après l'ammoniaque selon la revendication 1 est caractérisé de sorte que la couche sensible (4) de l'élément réactif, qui est formée en qualité de membrane semi-perméable hydrophobe, se compose de polyéthylène, de polytétrafluoroéthylène ou de polypropylène.

7. Système de test pour la détermination d'une activité enzymatique de bactéries après l'ammoniaque selon la revendication 1 est caractérisé de sorte que le substrat pour la détection d'une activité enzymatique de la couche sensible (4) de l'élément réactif présente de l'urée, de l'arginine ou de l'ornithine.

8. Système de test pour la détermination d'une activité enzymatique de bactéries après l'ammoniaque selon la revendication 1 est caractérisé de sorte que le revêtement de fixation refermable (9) est exécuté à partir d'un adhésif en une seule partie et que l'adhésif en une seule partie peut être du polyuréthane, de l'acétophénone ou du polyisobutylène.

9. Système de test pour la détermination d'une activité enzymatique de bactéries après l'ammoniaque selon la revendication 1 est caractérisé de sorte que la couche adhésive (5) à laquelle les surfaces en vis-à-vis de la sous-couche (1) et de la feuille fixant (6) sont reliées est exécutée grâce à l'adhésif en une seule partie et que l'adhésif en une seule partie peut être du polyuréthane, du polyacétate de vinyle, du l'acrylate d'isobornyle ou du polyisobutylène.

10. Système de test pour la détermination d'une activité enzymatique de bactéries après l'ammoniaque selon la revendication 1 est caractérisé de sorte que la feuille de couverture (9) est exécutée sur une des parties à l'aide du bord d'attaque arqué sous forme de languette.

11. Système de test pour la détermination d'une activité enzymatique de bactéries après l'ammoniaque selon la revendication 1 est caractérisé de sorte que la feuille de couverture (9) est fixée sur une partie opposée du bord d'attaque arqué à l'aide d'une couche d'adhésif thermofusible.

12. Système de test pour la détermination d'une activité enzymatique de bactéries après l'ammoniaque selon la revendication 1 est caractérisé de sorte que la feuille de couverture (9) est soit transparente soit présente une zone transparente et que la zone transparente correspond à cet égard à la taille de l'ouverture de la feuille fixant (6).

Fig. 1

Fig. 2

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- RU 2521201
- RU 2597777
- RU 2553548
- WO 2011112106 A
- US 2010028937 A
- WO 9319200 A
- US 20120094371 A
- RU 2630651 C9
- DE 69330515 T2
- EP 3009519 A

**In der Beschreibung aufgeführte Nicht-Patentliteratur**

- **BEKANNT.** Verfahren zur Bestimmung der Urease-Aktivität. *Urologie und Nephrologie,* 1997, vol. 4, 13-14